(19) **Europäisches Patentamt**
**European Patent Office**
**Office européen des brevets**

(11) **EP 3 897 381 B1**

(12) **EUROPEAN PATENT SPECIFICATION**

(45) Date of publication and mention
of the grant of the patent:
**12.04.2023 Bulletin 2023/15**

(21) Application number: **19818140.6**

(22) Date of filing: **17.12.2019**

(51) International Patent Classification (IPC):
**A61B 5/103** (2006.01) **A61B 5/11** (2006.01)
**A61B 5/00** (2006.01)

(52) Cooperative Patent Classification (CPC):
**A61B 5/1038; A61B 5/112; A61B 5/6807;**
A61B 2562/0247

(86) International application number:
**PCT/EP2019/085672**

(87) International publication number:
**WO 2020/127279 (25.06.2020 Gazette 2020/26)**

(54) **GAIT ANALYSIS DATA TREATMENT**

GANGANALYSEDATENBEHANDLUNG

TRAITEMENT DE DONNÉES D'ANALYSE DE DÉMARCHE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 LU 101071**

(43) Date of publication of application:
**27.10.2021 Bulletin 2021/43**

(73) Proprietor: **Luxembourg Institute of Science and
Technology (LIST)**
**4362 Esch-sur-Alzette (LU)**

(72) Inventor: **MELAKESSOU, Foued**
**57390 Russange (FR)**

(74) Representative: **Lecomte & Partners**
**76-78, rue de Merl**
**2146 Luxembourg (LU)**

(56) References cited:
**EP-A1- 2 783 630** **WO-A2-2015/164456**
**US-A1- 2003 163 287** **US-A1- 2017 020 445**
**US-A1- 2018 049 671**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention is directed to the field of human motion analysis, more particularly to a method and a system for analyzing the gait of a user.

## Technical field

The gait analysis is the systematic study of animal locomotion. The gait analysis is used to assess and treat individuals with conditions affecting their ability to walk. It is also commonly used in sports biomechanics to help athletes run more efficiently and to identify posture-related or movement-related problems in people with injuries. To calculate the kinetics of gait patterns, most labs have floor-mounted load transducers, which measure the ground reaction forces and moments.

In order to simplify the complex installation required for analyzing gait motion, an insole comprising pressure sensors has been developed. This wearable insole solution has been found to be particularly useful. The simplicity of the insole fitted with sensors and the development of smart devices such as smartphone make accessible such a technology to any users. Indeed, it could benefit the sportsman, people with mobility problem and people with any kind of physical, mobile or dynamic activities such as dancers to have their motions monitored outside a lab. However, the amount of data recorded for the analysis the gait poses a problem. Ordinary smart device does not have enough memory to record for instance an entire day of activity.

## Disclosure of the invention

The invention has for technical problem to provide a solution to at least one drawback of the above cited prior art. More specifically, the invention has for technical problem to provide a solution to improve the way to store the data related to the gait analysis.

For this purpose, the invention is directed to a method for analyzing the gait of a user according to claim 1 comprising the steps of: providing an article of footwear with a plurality of pressure sensors measuring the pressure applied thereon and generating output signals; providing an evaluation module with a transmitter, the evaluation module receiving the output signals of the pressure sensors; and providing a computing device with a receiver for receiving data transmitted by the evaluation module; the module being configured for: recording, during a plurality of stride cycles, the pressure applied to each pressure sensor to obtain a respective curve of pressure as a function of time for each pressure sensor, the respective curve being determined from the output signals; determining an initial time for each of the stride cycles on the basis of the pressure curves; segmenting each curve at each of the initial times; superimposing the segmented curves of at least one sensor ; computing stride key indicators based on the superimposed segmented curves; and transmitting the stride key indicators to the computing device.

The evaluation module is integrated into the article of footwear.

According to a preferred embodiment, the initial times are common to all the pressure curves and are determined by the time when the pressure and/or the pressure gradient changes from zero to a non-zero value on at least a first particular sensor, for instance the sensor that is positioned at the most rear position of the footwear.

According to a preferred embodiment, that the initial times are common to all the pressure curves and are determined by the time when at least a pressure recorded from the pressure curves changes from zero to a non-zero value and all other pressures remain equal to a zero value.

According to a preferred embodiment, the module is configured for determining a final time for each of the stride cycles on the basis of the pressure curves, the final times being common to all the pressure curves and being determined by the time when the pressure changes from non-zero to a zero value on at least a second particular sensor, for instance the sensor that is positioned at the most front position of the article of footwear.

According to a preferred embodiment, the module is configured for determining a final time for each of the stride cycles on the basis of the pressure curves, the final times being common to all the pressure curves and being determined by the time when at least a pressure from the pressure curves changes from a non-zero to a zero value and all other pressures remain equal to a zero value.

According to a preferred embodiment, the stride key indicators are determined individually for each sensor.

According to a preferred embodiment, the stride key indicators are based on the curves of pressure of all the sensors, preferably a weighted sum of the curves of pressure of all the sensors, more preferably a sum of the curves of pressure of all the sensors.

According to a preferred embodiment, the stride key indicators are selected from the group consisting of: the maximum pressure over a stride cycle, the average pressure over the stride cycle, the duration of the stride cycle, the duration of the stride cycle, the point in time when the pressure changes from a non-zero value to a zero value, the duration when the pressure curve is not equal to zero, the integral of the pressure over the duration of the stride cycle, a linear combination thereof.

According to a preferred embodiment, the stride key indicators further comprise the stand duration over a stride cycle.

According to a preferred embodiment, the position of the center of pressure is calculated at each moment in time based on the curves of pressure of all the sensors.

According to a preferred embodiment, the stride key indicators are further selected from the group consisting of: the distance travelled by the position of the center of pressure per cycle, the width of the path traveled by the center of pressure, the length of the path covered by the center of pressure, the average center of pressure per cycle, a linear combination thereof.

Acceptable values or ranges of values are defined for each stride key indicator and when one of the stride key indicators departs from its acceptable values or ranges of values, a signal is transmitted to the computing device.

The evaluation module is configured to generate a new key indicator based on the detected persistent variations of the curves prior or during the detection of a key indicator departing from its acceptable values or ranges of values.

According to a preferred embodiment, the evaluation module is configured to detect a change of gait based on the detected persistent variations of the curves prior or during the detection of a key indicator departing from its acceptable values or ranges of values.

According to a preferred embodiment, the acceptable values or ranges are preset or based on averaged or reference values of previous cycle.

According to a preferred embodiment, the stride key indicators are determined on a predetermined number of cycles, preferably 10 cycles or a temporal window, preferably amounting to 30 seconds.

According to a preferred embodiment, the stride key indicators are determined on a variable number of cycles, the variable number being dependent on the detected persistent variations of the curves prior or during the detection of a key indicator departing from its acceptable values or ranges of values.

According to a preferred embodiment, the recorded data are overwritten after the number of cycles is reached.

The evaluation module comprises a memory, a battery and a switching device, the switching device being configured for determining a remaining capacity of the memory and/or a level of charge of the battery; upon detection of the remaining capacity of the memory and/or the level of charge of the battery being below a predetermined threshold, a plurality of pressure curves of other stride cycles is transmitted to the computing device.

The computing device upon receiving the plurality of pressure curves of other stride cycles is configured for: recording the pressure applied to each pressure sensor to obtain a respective curve of pressure as a function of time for each pressure sensor, the respective curve being determined from the output signals; determining an initial time for each of the stride cycles on the basis of the curves; segmenting each curve at each of the initial times; superimposing the segmented curves of at least one sensor; computing the stride key indicators based on the superimposed segmented curves.

According to a preferred embodiment, a server is provided and the computing device is configured to transfer the data corresponding to the plurality of pressure curves of other stride cycles to the server.

According to a preferred embodiment, the computing device is a smart phone. According to a preferred embodiment, the pressure sensors are distributed over a portion of the article of footwear adapted to face the sole of a foot.

The article of footwear is a shoe, an insole, a sole of shoe or a sock.

The method further comprises the step of providing a further article of footwear with a plurality of further pressure sensors; measuring the pressure applied thereon and generating output signals, the further article of footwear being a shoe, an insole, a sock or a shoe sole; the module being configured for: recording, during a plurality of further stride cycles, the pressure applied to each of further sensors to obtain a respective curve of pressure as a function of time for each of further pressure sensors, the respective curve being determined from the output signals of the plurality of further pressure sensors ; determining an initial time for each of the further stride cycles on the basis of the pressure curves; segmenting each curve at each of the initial times; superimposing the segmented curves of at least one further sensor ; computing stride key indicators based on the superimposed segmented curves; and transmitting the stride key indicators to the computing device.

According to a preferred embodiment, the evaluation module is configured for computing step key indicators based on at least of the plurality of stride cycles and the plurality of further stride cycles.

According to a preferred embodiment, the evaluation module is configured for computing step key indicators based on at least of the plurality of stride cycles, the plurality of other stride cycles and the plurality of further stride cycles.

The invention is also directed to a system according to claim 14 for analyzing the gait of a user comprising: an article of footwear with a plurality of pressure sensors measuring the pressure applied thereon and generating output signals; an evaluation module with a transmitter, the evaluation module receiving the output signals of the pressure sensors ; and a computing device with a receiver for receiving data transmitted by the evaluation module; the evaluation module being configured for: recording, during a plurality of stride cycles, the pressure applied to each pressure sensor to obtain a respective curve of pressure as a function of time for each sensor, the respective curve being determined from the output signals; determining an initial time for each of the stride cycles on the basis of the curves; segmenting each curve at each of the initial times; superimposing the segmented curves of at least one sensor; computing stride key indicators based on the superimposed segmented curves; and transmitting stride the key indicators to the computing device.

The evaluation module is integrated into the article of footwear.

According to a preferred embodiment, the article of footwear comprises eight pressure sensors distributed over a portion of the article of footwear facing the sole of a foot.

According to a preferred embodiment, the pressure sensors are adapted to measure a pressure between 0.1 and 7 bars, preferably between 0.1 and 6 bars. According to a preferred embodiment, the article of footwear is a shoe, an insole, a sole of shoe or a sock.

The invention is interesting in that it provides a way to reduce the energy consumption of the gait evaluation unit by sharing the workload with the computation unit. The invention allows monitoring the gait for medical and sport purpose, wherein a practitioner or a trainer is directly informed by a change in the gait of patient or sportsman, respectively. The invention permits the miniaturization of the electronic components. The invention analyses the data recorded based on a leaning algorithm, improving the quality of the data computed.

## Brief description of the drawings

Other features and advantages of the present invention will be readily understood from the following detailed description and drawings among them:

- Figure 1 shows a sole with pressure sensors;
- Figure 2 depicts the superposition method applied to a plurality of pressure curves measured;
- Figure 3 represents the sum of a plurality of pressure curves measured;
- Figure 4 describes trajectories of the geometric center of pressure;

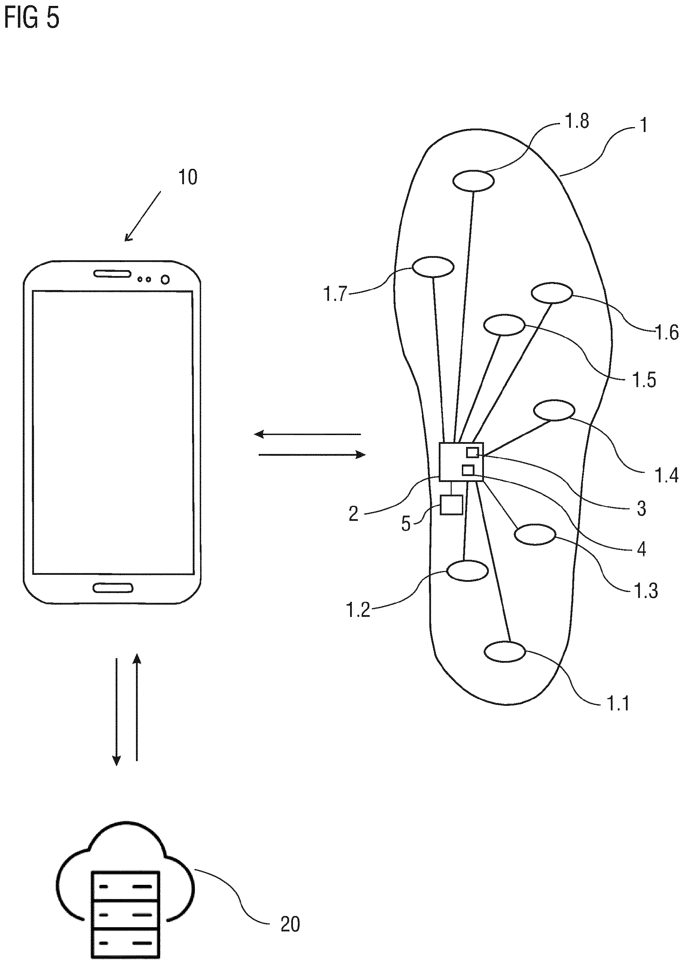

- Figure 5 shows an insole combined with a server;
- Figure 6 illustrates two insoles with a wireless connection whit a T-shirt.

## Detailed description

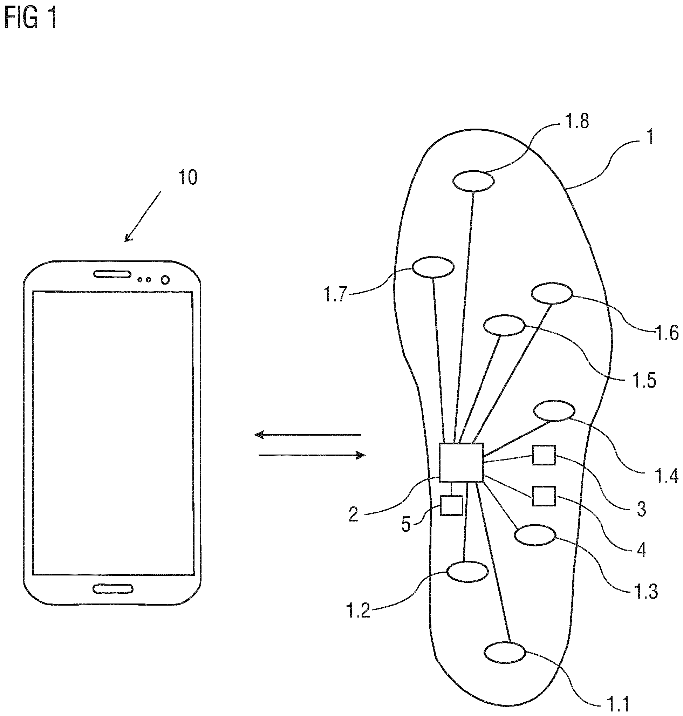

Figure 1 depicts an article of footwear 1, more precisely an insole 1 equipped with a plurality of pressure sensors 1.1, 1.2, 1.3, 1.4, 1.6, 1.7, 1.8. The invention is not limited to an insole 1. Indeed, a plurality of sensors can be provided on a sock, a shoe or a sole of a shoe for instance in order to record the pressure under the sole of a foot. In the present case the insole 1 comprises 8 sensors, but the number of sensors can vary depending on the needs.

The article of footwear 1 according to figure 1 comprises an evaluation module 2. In figure 1, the transmitter of the evaluation module 2 (data logger) can be connected to an (external) antenna 5 provided in the insole 1. Alternatively, the evaluation module 2 can comprise an internal antenna 5 (not shown). In another alternative, the transmitter can have wire connections to the computing device 10. The evaluation module 2 receives output signals of the plurality of pressure sensors 1.1, 1.2, 1.3, 1.4, 1.6, 1.7, 1.8. The evaluation module 2 is at least configured for recording the pressure applied to each pressure sensor 1.1, 1.2, 1.3, 1.4, 1.6, 1.7, 1.8 to obtain a respective curve of pressure as a function of time. For this embodiment, the evaluation module 2 is integrated in the insole 1 and connected to the sensors 1.1, 1.2, 1.3, 1.4, 1.6, 1.7, 1.8 via metallic wires. This arrangement allows short electric connections so that the user is not impeded. Preferably, the evaluation module 2 is clipped to a sock or shoe, the evaluation module 2 being connected to the sensors 1.1, 1.2, 1.3, 1.4, 1.6, 1.7, 1.8 of the insole 1 by wires. Alternatively, the communications between the plurality of sensors 1.1, 1.2, 1.3, 1.4, 1.6, 1.7, 1.8 and the evaluation unit 2 can be wireless.

The article of footwear 1 depicted in figure 1 also comprises a source of energy 3 such as a battery 3 for the supply of current to the evaluation unit 2 and the plurality of sensors 1.1, 1.2, 1.3, 1.4, 1.6, 1.7, 1.8. In an alternative embodiment, the battery 3 can be integrated into the evaluation unit 2. The source of energy 3 can be piezo energy using the compression on the insole 1 to generate electricity for the evaluation unit 2 and the plurality of sensors 1.1, 1.2, 1.3, 1.4, 1.6, 1.7, 1.8. Furthermore, the plurality of sensors 1.1, 1.2, 1.3, 1.4, 1.6, 1.7, 1.8 can be also piezo elements generating the electricity needed by the evaluation module 2.

The article of footwear can contain an external memory 4 to increase the memory capacity of the evaluation module 2.

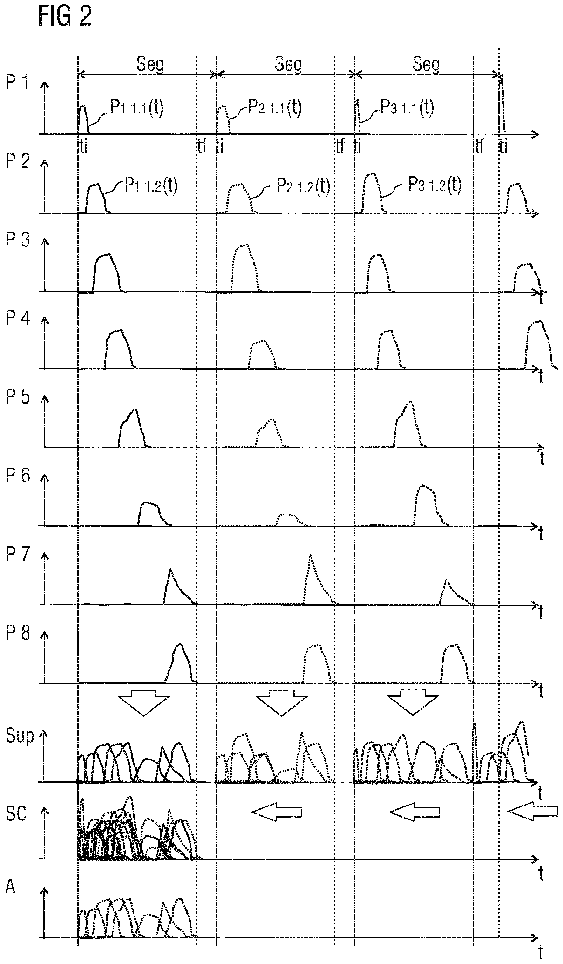

Figure 2 illustrates the data recorded by the plurality of sensors 1.1, 1.2, 1.3, 1.4, 1.6, 1.7, 1.8 mounted into the article of footwear 1. Figure 2 shows pressure curves, wherein one graph P1- P8 is displayed for each sensor. The x-axis corresponds to the time, while the y-axis shows the pressure recorded. The pressure curves can be periodic or quasi periodic and show cycles corresponding to the strides. For the quasi periodic cycle, the period can change from one cycle to another. A stride cycle starts, for instance during a walk or run, when a sensor positioned at the rear most position 1.1 of the insole 1 detects the contact of a shoe with the ground and ends when the same sensor 1.1 is pressed at the beginning of the next stride.

A stride cycle according to the invention also encompasses transient phase. For instance, a dancer stepping from the tip a foot activating the most front sensor 1.8 to a position where the dancer steps on a heel activating the most rear sensor 1.1. In this case, two transient cycles take place. In the first one, only the most front sensor 1.8 is activated, while all other sensors remain inactivate. In the second one, the most rear sensor 1.1 is activated, while all other sensors remain inactivate. The definition of a stride can be adapted to the final use, e.g. running, climbing, walking, classical dance.

Stride key indicators are determined by first segmenting the measured curves as shown in figure 2, where an initial time ti is determined for each stride cycle. The initial time ti can be detected when the pressure sensor 1.1 positioned on the rear most position change from a non-zero value to a zero value. The initial time ti for a stride can be defined as being the same for all pressures recorded as shown in figure 2. A segment Seg can be extracted for a given pressure curve. The segment Seg can start at the initial time ti and can end at the beginning of the initial time ti of the next stride cycle. This operation can be repeated for all the other output values, corresponding to the remaining N-1 segments Seg, N being the number of pressure sensors. All segments Seg can then be superposed on each other, as shown in figure 2 in graph Sup. The computation of the stride key indicators of the gait based on the superposed segmented curves serves as basis for the stride key indicator SKI, which can be calculated for each stride. In order to reduce the fluctuation from one cycle to another, a collection of pressure curves can be grouped and then averaged over several stride cycles as shown in figure 2 in graphs SC and A, respectively.

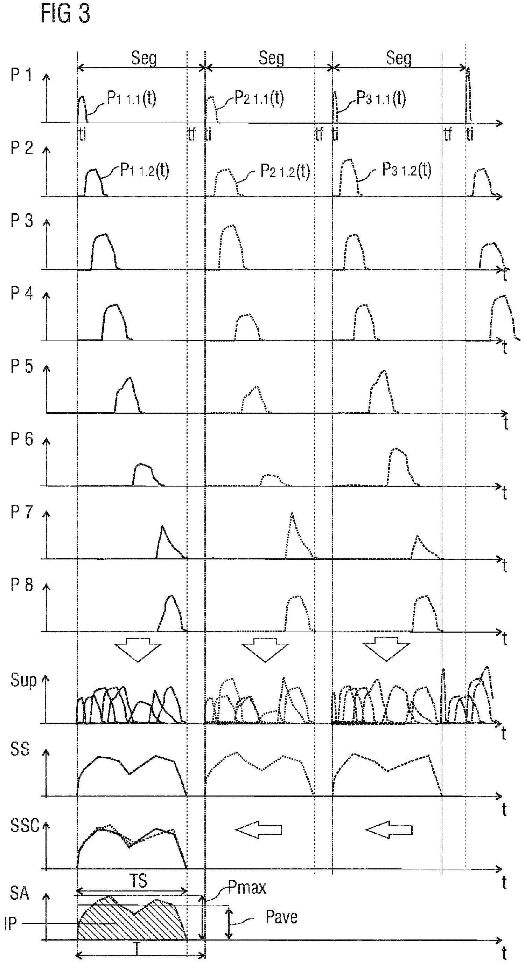

In a preferred embodiment, as shown in figure 3, the plurality of pressure curves can be summed, grouped, and optionally averaged as shown in figure on graphs SS, SSC and SA, respectively. The sum of the pressures curves is based on the following formula:

$$\mathrm{P}_i\left(\mathrm{t}\right) = \sum_{k=1}^{N} P_{i\,1.k}(t)$$

where:

P$_i$(t) corresponds to the sum of the superposed pressures curves measured for each sensor for stride cycle i;
Pi 1.k(t) corresponds to the segmented pressure curve recorded for the pressure sensor 1.k for the stride cycle i;
N is the number of sensors.

The stride key indicators SKI are selected from the group consisting of: the maximum pressure Pmax over a stride cycle, the average pressure Pave over the stride cycle, the duration T of the stride cycle, the point in time when the pressure changes from a non-zero value to a zero value, the duration during which the pressure curve is not equal to zero, the integral of the pressure IP over the duration of the stride cycle, as shown in figure 3.

The point in time when the pressure changes from non-zero to a zero value on sensor 1.8 at the most front position of the article of footwear 1 can correspond to the final time tf. The duration between the initial time ti and the final time tf is the stance duration TS of the stride cycle. The stance duration TS of a stride cycle can be a further stride key indicator SKI. The segment Seg can alternatively be defined as starting at the initial time ti and ending at the final time ft. Also, the swing duration for a cycle is the difference between the stride cycle duration T and the stance duration TS. The swing duration as well the ratio between the stance duration TS and the swing duration can be used as stride key indicators SKI.

In another preferred embodiment, a position of the center of pressure is calculated at each moment in time based on a linear combination (weighted sum) of the superposed pressure curves of the plurality of pressure sensors. The position of the center of pressure can be determined with the following formulas:

$$\mathrm{x}_{G\,i}\left(\mathrm{t}\right) = \frac{\sum_{k=1}^{N} x_{1.k}\, P_{i\,1.k}(t)}{\sum_{k=1}^{N} P_{i\,1.k}(t)}$$

$$y_{G\,i}\left(\mathrm{t}\right) = \frac{\sum_{k=1}^{N} y_{1.k}\, P_{i\,1.k}(t)}{\sum_{k=1}^{N} P_{i\,1.k}(t)}$$

where:

xG i (t) corresponds to the position of the geometric center of pressure G according to the x-axis for stride cycle i;
yG i (t) corresponds to the position of the geometric center of pressure G according to the y-axis for stride cycle i;
$x_{1.k}$ corresponds to the position of the pressure sensor 1.k according to the x-axis;
$y_{1.k}$ corresponds to the position of the pressure sensor 1 .k according to the y-axis;
P$_{i\,1.k}$(t) corresponds to the segmented pressure curve recorded for the pressure sensor 1.k for the stride cycle i;
N is the number of sensors.

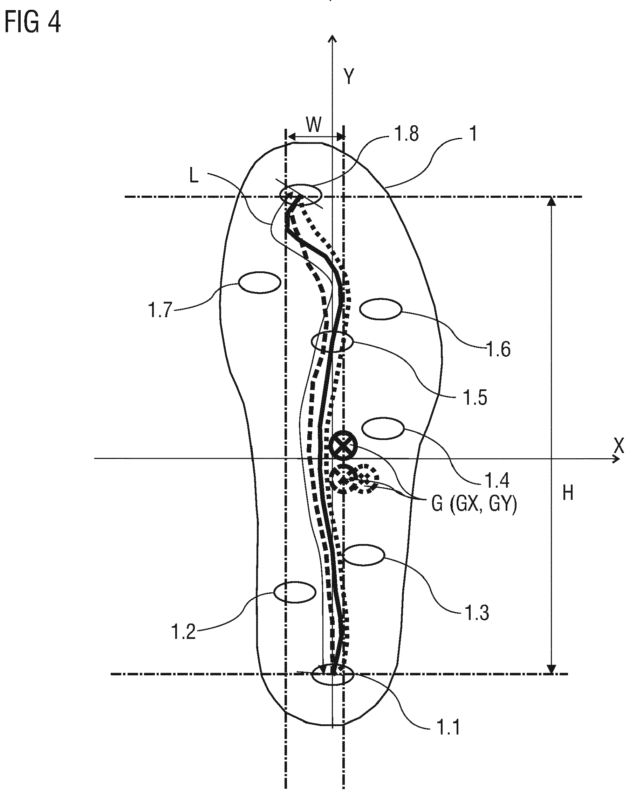

As shown if figure 4, the stride key indicators SKI are determined on the basis of the trajectory of the geometric center G selected from the group consisting of: the distance L travelled by the position of the geometric center of pressure G per cycle i, the width W of the path traveled by the center of pressure, the length H of the path covered by the center of pressure. The length H of the path traveled can change when the person just rests on the heel activating a part of the pressure sensors. In figure 4, the length and width W are determined for the continuous path line, which corresponds to a stride. This occurs during a transition phase for instance. Also, the average center of pressure is a further key indicator SKI and determined for each cycle with the coordinates (GX, GY), with the following formulas:

$$GX = \frac{\int_{ti}^{ti+T} \mathrm{x}_{G\,i}\left(\mathrm{t}\right) dt}{T}$$

$$GY = \frac{\int_{ti}^{ti+T} y_{G\,i}(t)dt}{T}$$

where:

xG i (t) corresponds to the position of the geometric center of pressure G according to the x-axis for stride cycle i;

yG i (t) corresponds to the position of the geometric center of pressure G according to the x-axis for stride cycle i;

T is the duration of a stride cycle (T can change from one stride cycle to another cycle). The trajectories of the geometric center of pressure can be averaged/smoothed to reduce the noise/fluctuation.

In a preferred embodiment, the stride key indicators SKI are monitored by the evaluation module 2. For instance, acceptable values or ranges of values are defined for each stride key indicator SKI. When one of the stride key indicators SKI departs from its acceptable values or ranges of values, a signal is transmitted to the computing device 10.

Furthermore, the evaluation module 2 is configured to generate a new key indicator SKI based on the detected persistent variations of the curves prior or during the detection of a stride key indicator SKI departing from its acceptable values or ranges of value. For instance, an evaluation module 2 is mounted on a shoe. In an initial phase, the pressures 1.1, 1.2, 1.3, 1.4, 1.6, 1.7, 1.8 are recorded by insole 1. Then, the user decides to wear a sock equipped with pressure sensors 1.1, 1.2, 1.3, 1.4, 1.5, 1.6, 1.7, 1.8 replacing the insole 1. The transition from one article of footwear to another one implies a change in the coordinates of the pressure sensors. This required an adaptation of the computation of stride key indicators SKI, because the relative distances between the sensors 1.1, 1.2, 1.3, 1.4, 1.5, 1.6, 1.7, 1.8 are altered.

The introduction of the stride key indicators SKI allows a significant reduction in the amount of information, simplifying the management of the memory and reducing the required memory capacity.

Equally, the evaluation module 2 is configured to detect a change of gait based on the detected persistent variations of the curves prior or during the detection of a key indicator departing from its acceptable values or ranges of value. For instance, a user abnormal gait can be detected following an injury of the user. This abnormality can generate an incident that is sent to a practitioner so that further actions can be taken. Equally, the acceptable values or ranges can be preset or be based on averages of previous cycle. For instance, a person climbing a mountain can be monitored. The comparison of the stride key indicators SKI between the beginning of the climb and the end for instance can reflect the tiredness of climber and/or the degree of the slope of the climb. Also, the comparison to the average of previous cycle, allows filtering for slow variations resulting for extrinsic perturbation and not resulting from a change in the gait.

The evaluation module 2 includes an internal memory 4. Optionally, an additional (external) memory 4 is foreseen to increase the memory capacity. The external memory 4 can be interchangeable. The monitoring of the gait generates a large amount of data and therefore needs an appropriate data management. For this purpose, the evaluation module 2 is configured to overwrite previously recorded data after a number of cycles is reached, for instance 10.

In another preferred embodiment, the computing device 10 can communicate with a remote server 20, which eases the management of the work load of the evaluation module 2. The server 20 can also store a large amount data that can be processed in due time. The server 20 can be configured to compute the key indicators SKI in the same way as the evaluation module 2 or the computing device does. In this configuration, the evaluation module 2 can comprise an internal memory 4 and a battery 3. The workload management is performed by a switching device (not shown) that is part of or external to the evaluation module 2. The switching device is configured for determining a remaining capacity of the memory 4 and/or a level of charge the battery 3. If it is detected that the capacity of the memory 4 and/or the level of charge of the battery 3 is below a predetermined threshold, the plurality of pressure outputs is not recorded in the memory 4 but is transmitted to the computing device 10. Once the computing device 10 receives the data corresponding to the plurality of pressure curves, the computing device 10 determines the stride key indicators SKI for these data. This approach allows discharging the evaluation modules 2. Also, the server 20 can be used to store the data.

The plurality of pressure sensors 1.1, 1.2, 1.3, 1.4, 1.6, 1.7, 1.8 are adapted to measure a pressure between 0.1 and 7 bars, preferably between 0 and 6 bars. The detection of a passage from non-zero value to a value or form a zero value to a non-zero value could be based on a minimal pressure detection threshold, preferably 0.1 bar. The minimal pressure detection threshold can correspond to the resolution of the pressure measurement, therefore amounting to 0.1 bar.

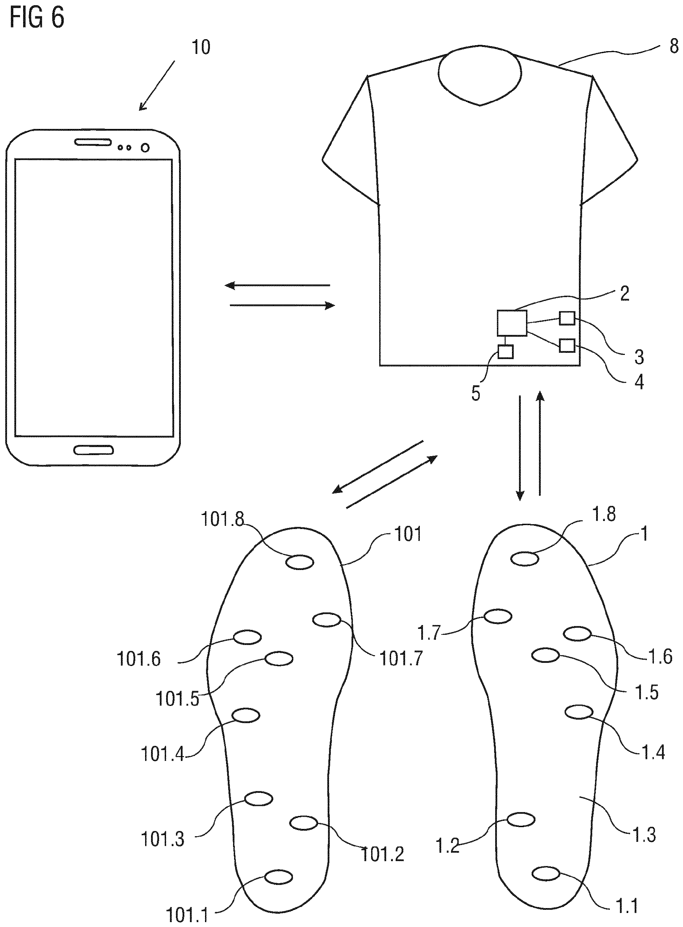

Figure 6 describes a further example not according to the present invention where the evaluation unit 2 records the pressure data from two separated articles of footwear such as two insoles 1, 101, the right and left respectively. In figure 6, the evaluation unit 2 is mounted on a garment 8 (T-shirt). In an alternative the evaluation unit 2 can be mounted on one of the insoles 1,101 so that a pair of interconnected insoles 1, 101 can be sold. Each of the pressure sensors 1.1, 1.2, 1.3, 1.4, 1.6, 1.7, 1.8, 101.1, 101.2, 101.3, 101.4, 101.6, 101.7, 101.8 of the article of footwear 1 and the further article of footwear 101 is provided with a suitable electric supply (not shown) such as a battery, piezo energy source and transmits to the evaluation unit 2 the pressure measurements via a wireless communication (not shown). With such configuration it is possible to determine for instance step key indicators such as the right or left step time.

The stride key indicators SKI can also be treated to represent relative values. For instance, the stand duration TS can be divided by the stride cycle T and the corresponding ratio is transmitted to the computation unit 2. In the case of a pair of insoles, the relative values allow comparing easily the key indicators of the two insoles.

**Claims**

1. Method for analyzing the gait of a user comprising the steps of:

   - providing a first article of footwear (1) with a plurality of first pressure sensors (1.1, 1.2, 1.3, 1.4, 1.5, 1.6, 1.7, 1.8) measuring the pressure applied thereon and generating output signals;
   - providing a second article of footwear (1) with a plurality of second pressure sensors (1.1, 1.2, 1.3, 1.4, 1.5, 1.6, 1.7, 1.8) measuring the pressure applied thereon and generating output signals, the first and second articles of footwear (1) being a shoe, an insole, a sock or a shoe sole;
   - providing an evaluation module (2) integrated in each article of footwear (1), the evaluation module having a memory (4), a battery (3), a switching device configured for determining a remaining capacity of the memory (4) and/or a level of charge the battery (3), as well as a transmitter, the evaluation module (2) receiving the output signals of the respective pressure sensors (1.1, 1.2, 1.3, 1.4, 1.5, 1.6, 1.7, 1.8); and
   - providing a computing device (10) with a receiver for receiving data transmitted by the evaluation module (2);
   wherein when the remaining capacity of the memory (4) and/or a level of charge the battery (3) of the evaluation module (2) is sufficient, the evaluation module (2) is configured for:

      - recording, during a plurality of stride cycles, the pressure applied to each pressure sensor (1.1, 1.2, 1.3, 1.4, 1.5, 1.6, 1.7, 1.8) to obtain a respective curve of pressure as a function of time for each pressure sensor (1.1, 1.2, 1.3, 1.4, 1.5, 1.6, 1.7, 1.8), the respective curve being determined from the output signals;
      - determining an initial time (ti) for each of the stride cycles on the basis of the pressure curves;
      - segmenting each curve at each of the initial times (ti);

      - superimposing the segmented curves of at least one sensor (1.1, 1.2, 1.3, 1.4, 1.5, 1.6, 1.7, 1.8);
      - computing stride key indicators (SKI) based on the superimposed segmented curves; and
      - transmitting the stride key indicators (SKI) to the computing device (10); and

   upon the switching device detecting that the remaining capacity of the memory (4) and/or a level of charge the battery (3) of the evaluation module (2) is below a predetermined threshold, the evaluation module (2) is configured for:

      - transmitting the pressure curves of stride cycles to the computing device (10); and the computing device (10) upon receiving the plurality of pressure curves of other stride cycles is configured for:

         - recording the respective curve of pressure as a function of time for each pressure sensor (1.1, 1.2, 1.3, 1.4, 1.5, 1.6, 1.7, 1.8);
         - determining an initial time (ti) for each of the stride cycles on the basis of the curves;
         - segmenting each curve at each of the initial times (ti);
         - superimposing the segmented curves of at least one sensor (1.1, 1.2, 1.3, 1.4, 1.5, 1.6, 1.7, 1.8); and
         - computing the stride key indicators (SKI) based on the superimposed segmented curves,

   wherein acceptable values or ranges of values are preset or defined based on average or reference values of previous cycle, for each stride key indicator (SKI) and when one of the stride key indicators (SKI) departs from its acceptable values or ranges of values, a signal is transmitted to the computing device (10), and
   wherein the evaluation module (2) is configured to generate a new key indicator based on the detected persistent variations of the curves prior or during the detection of a stride key indicator departing from its acceptable values or ranges of values.

2. Method according to claim 1, **characterized in that** the initial times (ti) are common to all the pressure curves and are determined by the time when the pressure and/or the pressure gradient changes from zero to a non-zero value on at least a first particular sensor (1.1, 1.2, 1.3, 1.4, 1.5, 1.6, 1.7, 1.8), for instance the sensor (1.1) that is positioned at the most rear position of the footwear; or the initial times (ti) are common to all the pressure curves and are determined by the time when at least a pressure recorded from the pressure curves changes from zero to a non-zero value and all other pressures remain equal to a zero value; and/or the method is **characterized in that** the evaluation module (2) is configured for determining a final time (tf) for each of the stride cycles on the basis of the pressure curves, the final times (tf) being common to all the pressure curves and being determined by the time when the pressure changes from non-zero to a zero value on at least a second particular sensor (1.1, 1.2, 1.3, 1.4, 1.5, 1.6, 1.7, 1.8), for instance the sensor (1.8) that is positioned at the most front position of the article of footwear; or the final times being determined by the time when at least a pressure from the pressure curves changes from a non-zero to a zero value and all other pressures remain equal to a zero value .

3. Method according to claim 1 or 2, **characterized in that** the stride key indicators (SKI) are determined individually for each sensor (1.1, 1.2, 1.3, 1.4, 1.5, 1.6, 1.7, 1.8).

4. Method according to any of claims 1 to 3, **characterized in that** the stride key indicators (SKI) are based on the curves of pressure of all the sensors (1.1, 1.2, 1.3, 1.4, 1.5, 1.6, 1.7, 1.8), preferably a weighted sum of the curves of pressure of all the sensors (1.1, 1.2, 1.3, 1.4, 1.5, 1.6, 1.7, 1.8), more preferably a sum of the curves of pressure of all the sensors (1.1, 1.2, 1.3, 1.4, 1.5, 1.6, 1.7, 1.8).

5. Method according to any of claims 1 to 4, **characterized in that** the stride key indicators (SKI) are selected from the group consisting of: the maximum pressure (Pmax) during a stride cycle, the average pressure (Pave) over the stride cycle, the duration of the stride cycle (T), the point in time when the pressure changes from a non-zero value to a zero value, the duration during which the pressure curve is not equal to zero, the integral of the pressure over the duration of the cycle (IP), a linear combination thereof; and/or a key indicator (SKI) is the stand duration (TS) over a stride cycle.

6. Method according to any of claims 1 to 5, **characterized in that** the position of the center of pressure is calculated at each moment in time based on the curves of pressure of all the sensors (1.1, 1.2, 1.3, 1.4, 1.5, 1.6, 1.7, 1.8) and the stride key indicators (SKI) are further selected from the group consisting of: the distance (L) travelled by the position of the center of pressure per cycle, the width (W) of the path traveled by the center of pressure, the length (H) of the path covered by the center of pressure, the average center of pressure per cycle (GX, GY), a linear combination thereof.

7. Method according to any of claims 1-6, **characterized in that** the evaluation module (2) is configured to detect a change of gait based on the detected persistent variations of the curves prior or during the detection of a key indicator departing from its acceptable values or ranges of values.

8. Method according to any of the claims 1 to 7, **characterized in that** the computing device (10) is a smart phone.

9. Method according to any of claims 1-8, **characterized in that** the evaluation module (2) is configured for computing the key indicators based on at least the plurality of stride cycles.

10. System for analyzing the gait of a user comprising:

- a first and a second article of footwear with a plurality of first and second pressure sensors (1.1, 1.2, 1.3, 1.4, 1.5, 1.6, 1.7, 1.8) measuring the pressure applied thereon and generating output signals;
- a respective evaluation module (2) integrated in each article of footwear (1), the evaluation module having a memory (4), a battery (3), a switching device configured for determining a remaining capacity of the memory (4) and/or a level of charge the battery (3), as well as a transmitter, the evaluation module (2) receiving the output signals of the pressure sensors (1.1, 1.2, 1.3, 1.4, 1.5, 1.6, 1.7, 1.8); and
- a computing device (10) with a receiver for receiving data transmitted by the evaluation module (2);
wherein when the remaining capacity of the memory (4) and/or a level of charge the battery (3) of the evaluation module (2) is sufficient, the evaluation module (2) is configured for:

- recording, during a plurality of stride cycles, the pressure applied to each pressure sensor (1.1, 1.2, 1.3, 1.4, 1.5, 1.6, 1.7, 1.8) to obtain a respective curve of pressure as a function of time for each sensor (1.1, 1.2, 1.3, 1.4, 1.5, 1.6, 1.7, 1.8), the respective curve being determined from the output signals;
- determining an initial time (ti) for each of the stride cycles on the basis of the curves;
- segmenting each curve at each of the initial times (ti);
- superimposing the segmented curves of at least one sensor (1.1, 1.2, 1.3, 1.4, 1.5, 1.6, 1.7, 1.8);
- computing stride key indicators (SKI) based on the superimposed segmented curves; and
- transmitting stride key indicators (SKI) to the computing device (10); and

upon the switching device detecting that the remaining capacity of the memory (4) and/or a level of charge the battery (3) of the evaluation module (2) is below a predetermined threshold, the evaluation module (2) is configured for:

- transmitting the pressure curves of stride cycles to the computing device (10); and the computing device (10) upon receiving the plurality of pressure curves of other stride cycles is configured for:

- recording the respective curve of pressure as a function of time for each pressure sensor (1.1, 1.2, 1.3, 1.4, 1.5, 1.6, 1.7, 1.8);
- determining an initial time (ti) for each of the stride cycles on the basis of the curves;
- segmenting each curve at each of the initial times (ti);
- superimposing the segmented curves of at least one sensor (1.1, 1.2, 1.3, 1.4, 1.5, 1.6, 1.7, 1.8); and
- computing the stride key indicators (SKI) based on the superimposed segmented curves,

wherein acceptable values or ranges of values are preset or defined based on average or reference values of previous cycle, for each stride key indicator (SKI) and when one of the stride key indicators (SKI) departs from its acceptable values or ranges of values, a signal is transmitted to the computing device (10), and wherein the evaluation module (2) is configured to generate a new key indicator based on the detected persistent variations of the curves prior or during the detection of a stride key indicator departing from its acceptable values or ranges of values.

11. System according to claim 10, **characterized in that** each article of footwear (1) comprises eight pressure sensors (1.1, 1.2, 1.3, 1.4, 1.5, 1.6, 1.7, 1.8) distributed over a portion of the article of footwear facing the sole of a foot and adapted to measure any pressure comprised between 0.1 and 7 bars.

**Patentansprüche**

1. Verfahren zur Analyse des Gangs eines Benutzers, das die folgenden Schritte umfasst:

- Bereitstellen eines ersten Schuhwerks (1) mit einer Vielzahl von ersten Drucksensoren (1.1, 1.2, 1.3, 1.4, 1.5, 1.6, 1.7, 1.8), die den auf sie ausgeübten Druck messen und Ausgangssignale erzeugen;
- Bereitstellen eines zweiten Schuhwerks (1) mit einer Vielzahl von zweiten Drucksensoren (1.1, 1.2, 1.3, 1.4, 1.5, 1.6, 1.7, 1.8), die den darauf ausgeübten Druck messen und Ausgangssignale erzeugen, wobei das erste und zweite Schuhwerk (1) ein Schuh, eine Einlegesohle, eine Socke oder eine Schuhsohle ist;
- Bereitstellen eines in jedem Schuhwerk (1) eingebauten Auswertemoduls (2), wobei das Auswertemodul einen Speicher (4), eine Batterie (3), eine Schalteinrichtung zur Ermittlung einer verbleibenden Kapazität des Speichers (4) und/oder eines Ladezustandes der Batterie (3) sowie einen Sender aufweist, wobei das Auswertemodul (2) die Ausgangssignale der jeweiligen Drucksensoren (1.1, 1.2, 1.3, 1.4, 1.5, 1.6, 1.7, 1.8) empfängt; und
- Bereitstellen einer Rechenvorrichtung (10) mit einem Empfänger zum Aufnehmen von Daten, die von dem Auswertemodul (2) übertragen werden;
wobei, wenn die verbleibende Kapazität des Speichers (4) und/oder ein Ladezustand der Batterie (3) des Auswertemoduls (2) ausreichend ist, das Auswertemodul (2) dazu ausgelegt ist:

- Aufzeichnen des auf jeden Drucksensor (1.1, 1.2, 1.3, 1.4, 1.5, 1.6, 1.7, 1.8) ausgeübten Drucks während einer Vielzahl von Schrittzyklen, um für jeden Drucksensor (1.1, 1.2, 1.3, 1.4, 1.5, 1.6, 1.7, 1.8) eine jeweilige Kurve des Drucks als Funktion der Zeit zu erhalten, wobei die jeweilige Kurve aus den Ausgangssignalen bestimmt wird;
- Bestimmen einer Anfangszeit (ti) für jeden der Schrittzyklen anhand der Druckkurven:

- Aufteilung jeder Kurve zu jedem der Anfangszeitpunkte (ti);
- Überlagern der Segmentkurven von mindestens einem Sensor (1.1, 1.2, 1.3, 1.4, 1.5, 1.6, 1.7, 1.8);
- Berechnen von Schrittschlüsselindikatoren (SKI) anhand der überlagerten Segmentkurven; und
- Übertragen der Schrittschlüsselindikatoren (SKI) an die Rechenvorrichtung (10); und wenn die Schaltvorrichtung feststellt, dass die verbleibende Kapazität des Speichers (4) und/oder ein Ladezustand der Batterie (3) des Auswertemoduls (2) unter einem vorbestimmten Schwellenwert liegt, ist das Auswertemodul (2) so ausgelegt dass:

- Übertragung der Druckkurven von Schrittzyklen an die Rechenvorrichtung (10); und die Rechenvorrichtung (10) ist beim Empfang der Vielzahl von Druckkurven anderer Schrittzyklen konfiguriert, um:

- Aufzeichnung der jeweiligen Druckkurve als Funktion der Zeit für jeden Drucksensor (1.1, 1.2, 1.3, 1.4, 1.5, 1.6, 1.7, 1.8);
- Bestimmen einer Anfangszeit (ti) für jeden der Schrittzyklen anhand der Kurven;
- Aufteilung jeder Kurve zu jedem der Anfangszeitpunkte (ti);
- Überlagern der Segmentkurven von mindestens einem Sensor (1.1, 1.2, 1.3, 1.4, 1.5, 1.6, 1.7, 1.8); und
- Berechnen der Schrittschlüsselindikatoren (SKI) anhand der überlagerten Segmentkurven,

wobei für jeden Schrittschlüsselindikator (SKI) zulässige Werte oder Wertebereiche anhand von Durchschnitts- oder Referenzwerten des vorhergehenden Zyklus voreingestellt oder definiert sind, und wenn einer der Schrittschlüsselindikatoren (SKI) von seinen zulässigen Werten oder Wertebereichen abweicht, ein Signal an die Rechenvorrichtung (10) übertragen wird, und wobei das Auswertemodul (2) so konfiguriert ist, dass es einen neuen Schlüsselindikator anhand der erfassten anhaltenden Schwankungen der Kurven vor oder während der Erfassung eines Schrittschlüsselindikators, der von seinen zulässigen Werten oder Wertebereichen abweicht, erzeugt.

2.  Verfahren nach Anspruch 1, **dadurch gekennzeichnet, dass** die Anfangszeiten (ti) allen Druckkurven gleich sind und durch den Zeitpunkt bestimmt werden, zu dem der Druck und/oder der Druckgradient an mindestens einem ersten bestimmten Sensor (1.1, 1.2, 1.3, 1.4, 1.5, 1.6, 1.7, 1.8), beispielsweise dem Sensor (1.1), der sich an der hintersten Position des Schuhs befindet, von Null auf einen Wert ungleich Null wechselt: oder die Anfangszeiten (ti) sind gleich für alle Druckkurven und werden durch den Zeitpunkt bestimmt, an dem mindestens ein von den Druckkurven aufgezeichneter Druck von Null auf einen Wert ungleich Null wechselt und alle anderen Drücke gleich Null bleiben;

und/oder das Verfahren **dadurch gekennzeichnet ist, dass** das Auswertemodul (2) dazu ausgebildet ist, für jeden der Schrittzyklen anhand der Druckkurven eine Endzeit (tf) zu bestimmen, wobei die Endzeiten (tf) allen Druckkurven gemeinsam sind und durch den Zeitpunkt bestimmt werden, an dem der Druck an mindestens einem zweiten bestimmten Sensor (1.1, 1.2, 1.3, 1.4, 1.5, 1.6, 1.7, 1.8), beispielsweise an dem Sensor (1.8), der sich an der vordersten Position des Schuhwerks befindet, von einem Wert ungleich Null auf einen Wert Null wechselt; oder die Endzeiten werden durch den Zeitpunkt bestimmt, zu dem mindestens ein Druck aus den Druckkurven von einem Wert ungleich Null auf einen Wert Null wechselt und alle anderen Drücke gleich einem Wert Null bleiben.

3.  Verfahren nach Anspruch 1 oder 2, **dadurch gekennzeichnet, dass** die Schrittschlüsselindikatoren (SKI) individuell für jeden Sensor (1.1, 1.2, 1.3, 1.4, 1.5, 1.6, 1.7, 1.8) bestimmt werden.

4.  Verfahren nach einem der Ansprüche 1 bis 3, **dadurch gekennzeichnet, dass** die Schrittschlüsselindikatoren (SKI) auf den Druckkurven aller Sensoren (1.1, 1.2, 1.3, 1.4, 1.5, 1.6, 1.7, 1. 8), vorzugsweise einer gewichteten Summe der Druckkurven aller Sensoren (1.1, 1.2, 1.3, 1.4, 1.5, 1.6, 1.7, 1.8), noch bevorzugter einer Summe der Druckkurven aller Sensoren (1.1, 1.2, 1.3, 1.4, 1.5, 1.6, 1.7, 1.8) basieren.

5.  Verfahren nach einem der Ansprüche 1 bis 4, **dadurch gekennzeichnet, dass** die Schrittschlüsselindikatoren (SKI) aus der folgenden Gruppe ausgewählt werden: dem maximalen Druck (Pmax) während eines Schrittzyklus, dem durchschnittlichen Druck (Pave) über den Schrittzyklus, der Dauer des Schrittzyklus (T), dem Zeitpunkt, zu dem der Druck von einem Wert ungleich Null auf einen Nullwert wechselt, der Dauer, während der die Druckkurve ungleich Null ist, dem Integral des Drucks über die Dauer des Zyklus (IP), einer linearen Kombination davon; und/oder ein Schlüsselindikator (SKI) die Standdauer (TS) über einen Schrittzyklus ist.

6.  Verfahren nach einem der Ansprüche 1 bis 5, **dadurch gekennzeichnet, dass** die Position des Druckzentrums zu jedem Zeitpunkt anhand der Druckkurven aller Sensoren (1.1, 1.2, 1.3, 1.4, 1.5, 1.6, 1.7, 1. 8) und die Schrittschlüsselindikatoren (SKI) sind ferner ausgewählt aus der Gruppe bestehend aus: der von der Position des Druckmittelpunkts pro Zyklus zurückgelegten Strecke (L), der Breite (W) des vom Druckmittelpunkt zurückgelegten Weges, der Länge (H) des vom Druckmittelpunkt zurückgelegten Weges, dem durchschnittlichen Druckmittelpunkt pro Zyklus (GX, GY), einer linearen Kombination davon.

7. Verfahren nach einem der Ansprüche 1 bis 6, **dadurch gekennzeichnet, dass** das Auswertemodul (2) so ausgelegt ist, dass es auf der Grundlage der erfassten anhaltenden Veränderungen der Kurven vor oder während der Erfassung eines Schlüsselindikators, der von seinen zulässigen Werten oder Wertebereichen abweicht, eine Veränderung der Gangart erkennt.

8. Verfahren nach einem der Ansprüche 1 bis 7, **dadurch gekennzeichnet, dass** die Rechenvorrichtung (10) ein Smartphone ist.

9. Verfahren nach einem der Ansprüche 1 bis 8, **dadurch gekennzeichnet, dass** das Auswertemodul (2) so ausgestaltet ist, dass es die Schlüsselindikatoren anhand von mindestens der Mehrzahl von Schrittzyklen berechnet.

10. System zur Analyse des Gangs eines Benutzers, das Folgendes umfasst:

- ein erstes und ein zweites Schuhwerk mit einer Vielzahl von ersten und zweiten Drucksensoren (1.1, 1.2, 1.3, 1.4, 1.5, 1.6, 1.7, 1.8), die den darauf ausgeübten Druck messen und Ausgangssignale erzeugen:
- ein in jedem Schuhwerk (1) eingebautes Auswertemodul (2), wobei das Auswertemodul einen Speicher (4), eine Batterie (3), eine Schalteinrichtung, die zur Bestimmung einer verbleibenden Kapazität des Speichers (4) und/oder eines Ladezustandes der Batterie (3) ausgebildet ist, sowie einen Sender aufweist, wobei das Auswertemodul (2) die Ausgangssignale der Drucksensoren (1.1, 1.2, 1.3, 1.4, 1.5, 1.6, 1.7, 1.8) empfängt; und
- eine Rechenvorrichtung (10) mit einem Empfänger zum Empfangen von durch das Auswertemodul (2) übertragenen Daten; wobei das Auswertemodul (2), wenn die verbleibende Kapazität des Speichers (4) und/oder ein Ladezustand der Batterie (3) des Auswertemoduls (2) ausreichend ist, konfiguriert ist zum:

- Aufzeichnen des auf jeden Drucksensor (1.1, 1.2, 1.3, 1.4, 1.5, 1.6, 1.7, 1.8) ausgeübten Drucks während einer Vielzahl von Schrittzyklen, um für jeden Sensor (1.1, 1.2, 1.3, 1.4, 1.5, 1.6, 1.7, 1.8) eine jeweilige Kurve des Drucks als Funktion der Zeit zu erhalten, wobei die jeweilige Kurve aus den Ausgangssignalen bestimmt wird;
- Bestimmen einer Anfangszeit (ti) für jeden der Schrittzyklen anhand der Kurven;
- Segmentierung jeder Kurve zu jedem der Anfangszeitpunkte (ti);
- Überlagern der Segmentkurven von mindestens einem Sensor (1.1, 1.2, 1.3, 1.4, 1.5, 1.6, 1.7, 1.8);
- Berechnen von Schrittschlüsselindikatoren (SKI) anhand der überlagerten Segmentkurven; und
- Übertragen von Schrittschlüsselindikatoren (SKI) an die Rechenvorrichtung (10); und wenn die Schaltvorrichtung feststellt, dass die verbleibende Kapazität des Speichers (4) und/oder ein Ladezustand der Batterie (3) des Auswertemoduls (2) unter einem vorbestimmten Schwellenwert liegt, ist das Auswertemodul (2) dazu konfiguriert:

- Übertragen der Druckkurven von Schrittzyklen an die Rechenvorrichtung (10); und die Rechenvorrichtung (10) ist beim Empfang der Vielzahl von Druckkurven anderer Schrittzyklen konfiguriert zum:

- Aufzeichnung der jeweiligen Druckkurve als Funktion der Zeit für jeden Drucksensor (1.1, 1.2, 1.3, 1.4, 1.5, 1.6, 1.7, 1.8);
- Bestimmen einer Anfangszeit (ti) für jeden der Schrittzyklen auf der Grundlage der Kurven;
- Segmentierung jeder Kurve zu jedem der Anfangszeitpunkte (ti);
- Überlagern der Segmentkurven von mindestens einem Sensor (1.1, 1.2, 1.3, 1.4, 1.5, 1.6, 1.7, 1.8); und
- Berechnen der Schrittschlüsselindikatoren (SKI) anhand der überlagerten Segmentkurven,

wobei für jeden Schrittschlüsselindikator (SKI) zulässige Werte oder Wertebereiche auf der Grundlage von Durchschnitts- oder Referenzwerten des vorherigen Zyklus voreingestellt oder definiert sind, und wenn einer der Schrittschlüsselindikatoren (SKI) von seinen zulässigen Werten oder Wertebereichen abweicht, ein Signal an die Rechenvorrichtung (10) übertragen wird, und

wobei das Auswertemodul (2) so konfiguriert ist, dass es einen neuen Schlüsselindikator auf der Grundlage der erfassten anhaltenden Schwankungen der Kurven vor oder während der Erfassung eines Schrittschlüsselindikators, der von seinen akzeptablen Werten oder Wertebereichen abweicht, erzeugt.

11. System nach Anspruch 10, **dadurch gekennzeichnet, dass** jedes Schuhwerk (1) acht Drucksensoren (1.1, 1.2, 1.3, 1.4, 1.5, 16, 1.7, 1.8) umfasst, die über einen der Fußsohle zugewandten Teil des Schuhwerks verteilt sind und jeden Druck zwischen 0,1 und 7 bar messen können.

**Revendications**

1. Procédé d'analyse de la démarche d'un utilisateur comprenant les étapes consistant à :

- fournir un premier article de chaussure (1) d'une pluralité de premiers capteurs de pression (1.1, 1.2, 1.3, 1.4, 1.5, 1.6, 1.7, 1.8) mesurant la pression qui y est appliquée et générant des signaux de sortie ;
- fournir un second article de chaussure (1) d'une pluralité de seconds capteurs de pression (1.1, 1.2, 1.3, 1.4, 1.5, 1.6, 1.7, 1.8) mesurant la pression appliquée sur celuici et générant des signaux de sortie, les premier et second articles de chaussure (1) étant une chaussure, une semelle intérieure, une chaussette ou une semelle de chaussure ;
- fournir un module d'évaluation (2) intégré dans chaque article chaussure (1), le module d'évaluation ayant une mémoire (4), une batterie (3), un dispositif de commutation configuré pour déterminer une capacité restante de la mémoire (4) et/ou un niveau de charge de la batterie (3), ainsi qu'un émetteur, le module d'évaluation (2) recevant les signaux de sortie des capteurs de pression respectifs (1.1, 1.2, 1.3, 1.4, 1.5, 1.6, 1.7, 1.8) ; et
- fournir un dispositif de calcul ( 10) avec un récepteur pour recevoir les données transmises par le module d'évaluation (2) ;

dans lequel, lorsque la capacité restante de la mémoire (4) et/ou un niveau de charge de la batterie (3) du module d'évaluation (2) est suffisant, le module d'évaluation (2) étant configuré pour :

- enregistrer, au cours d'une pluralité de cycles d'enjambée, la pression appliquée à chaque capteur de pression (1.1, 1.2, 1.3, 1.4, 1.5, 1.6, 1.7, 1.8) pour obtenir une courbe correspondante de pression en fonction du temps pour chaque capteur de pression (1.1, 1.2, 1.3, 1.4, 1.5, 1.6, 1.7, 1.8), la courbe correspondante étant déterminée à partir des signaux de sortie ;
- déterminer un temps initial (ti) pour chacun des cycles d'enjambée sur la base des courbes de pression ;
- segmenter chaque courbe à chacun des temps initiaux (ti) ;
- superposer les courbes segmentées d'au moins un capteur (1.1, 1.2, 1.3, 1.4, 1.5, 1.6, 1.7, 1.8) ;
- calculer des indicateurs clés d'enjambée (SKI) basés sur les courbes segmentées superposées ; et
- transmettre les indicateurs clés d'enjambée (SKI) au dispositif informatique (10) ; et

lorsque le dispositif de commutation détecte que la capacité restante de la mémoire (4) et/ou un niveau de charge de la batterie (3) du module d'évaluation (2) est inférieur à un seuil prédéterminé, le module d'évaluation (2) est configuré pour :

- transmettre les courbes de pression des cycles d'enjambée au dispositif informatique (10) ; et le dispositif de calcul (10), à la réception de la pluralité de courbes de pression de cycles d'enjambée supplémentaires, est configuré pour :

- enregistrer de la courbe correspondante de pression en fonction du temps pour chaque
capteur de pression (1.1, 1.2, 1.3, 1.4, 1.5, 1.6, 1.7, 1.8) ;
- déterminer un temps initial (ti) pour chacun des cycles d'enjambée sur la base des courbes ;
- segmenter chaque courbe à chacun des temps initiaux (ti) ;
- superposer les courbes segmentées d'au moins un capteur (1.1, 1.2, 1.3, 1.4, 1.5, 1.6, 1.7, 1.8) ; et
- calculer les indicateurs clés d'enjambée (SKI) sur la base des courbes segmentées superposées,

dans lequel les valeurs ou plages de valeurs acceptables sont préétablies ou définies sur la base de valeurs moyennes ou de référence du cycle précédent, pour chaque indicateur clé d'enjambée (SKI), et lorsque l'un des indicateurs clés d'enjambée (SKI) s'écarte de ses valeurs ou plages de valeurs acceptables, un message d'avertissement s'affiche, valeurs ou gammes de valeurs acceptables, un signal est transmis au dispositif informatique dispositif informatique (10), et

dans lequel le module d'évaluation (2) est configuré pour générer un nouvel indicateur clé sur la base des variations persistantes détectées de l'indicateur clé. indicateur clé basé sur les variations persistantes détectées des courbes avant ou pendant la détection d'un indicateur clé d'enjambée s'écartant de ses valeurs ou plages de valeurs acceptables.

2. Procédé selon la revendication 1, **caractérisé en ce que** les temps initiaux (ti) sont communs à toutes les courbes de pression et sont déterminés par le moment où la pression et/ou le gradient de pression passe de zéro à une valeur non nulle sur au moins un premier capteur particulier (1.1, 1.2, 1.3, 1.4, 1.5, 1.6, 1.7, 1.8), par exemple le capteur (1.1) qui est positionné à la position la plus arrière de la chaussure ; ou les temps initiaux (ti) sont communs à toutes les courbes de pression et sont déterminés par le temps où au moins une pression enregistrée à partir des courbes de pression passe de zéro à une valeur non nulle et toutes les autres pressions restent égales à une valeur nulle ; et/ou le procédé est **caractérisé en ce que** le module d'évaluation (2) est configuré pour déterminer un temps final (tf) pour chacun des cycles d'enjambée sur la base des courbes de pression, les temps finaux (tf) étant communs à toutes les courbes de pression et étant déterminés par le moment où la pression passe d'une valeur non nulle à une valeur nulle sur au moins un deuxième capteur particulier (1.1, 1.2, 1.3, 1.4, 1.5, 1.6, 1.7, 1.8), par exemple le capteur (1.8) qui est positionné à la position la plus avant de l'article de chaussure ; ou les temps finaux étant déterminés par le moment où au moins une pression provenant des courbes de pression passe d'une valeur non nulle à une valeur nulle et toutes les autres pressions restent égales à une valeur nulle.

3. Procédé selon la revendication 1 ou 2, **caractérisé en ce que** les indicateurs clés d'enjambée (SKI) sont déterminés individuellement pour chaque capteur (1.1, 1.2, 1.3, 1.4, 1.5, 1.6, 1.7, 1.8).

4. Procédé selon l'une quelconque des revendications 1 à 3, **caractérisé en ce que** les indicateurs clés d'enjambée (SKI) sont basés sur les courbes de pression de tous les capteurs (1.1, 1.2, 1.3, 1.4, 1.5, 1.6, 1.7, 1. 8), de préférence une somme pondérée des courbes de pression de tous les capteurs (1.1, 1.2, 1.3, 1.4, 1.5, 1.6, 1.7, 1.8), plus préférablement une somme des courbes de pression de tous les capteurs (1.1, 1.2, 1.3, 1.4, 1.5, 1.6, 1.7, 1.8).

5. Procédé selon l'une quelconque des revendications 1 à 4, **caractérisé en ce que** les indicateurs clés d'enjambée (SKI) sont choisis dans le groupe constitué par : la pression maximale (Pmax) pendant un cycle d'enjambée, la pression moyenne (Pave) sur le cycle d'enjambée, la durée du cycle d'enjambée (T), le moment où la pression passe d'une valeur non nulle à une valeur nulle, la durée pendant laquelle la courbe de pression n'est pas égale à zéro, l'intégrale de la pression sur la durée du cycle (IP), une combinaison linéaire de ceux-ci ; et/ou un indicateur clé (SKI) est la durée de station (TS) sur un cycle d'enjambée (SKI) est la durée de station debout (TS) sur un cycle d'enjambée.

6. Procédé selon l'une quelconque des revendications 1 à 5, **caractérisé en ce que** la position du centre de pression est calculée à chaque instant sur la base des courbes de pression de tous les capteurs (1.1, 1.2, 1.3, 1.4, 1.5, 1.6, 1.7, 1. 8) et les indicateurs clés d'enjambée (SKI) sont en outre choisis dans le groupe constitué par : la distance (L) parcourue par la position du centre de pression par cycle, la largeur (W) du chemin parcouru par le centre de pression, la longueur (H) du chemin parcouru par le centre de pression, le centre de pression moyen par cycle (GX, GY), une combinaison linéaire de ceux-ci.

7. Procédé selon l'une quelconque des revendications 1 à 6, **caractérisé en ce que** le module d'évaluation (2) est configuré pour détecter un changement de démarche sur la base des variations persistantes des courbes détectées avant ou pendant la détection d'une clé de lecture. des variations persistantes des courbes avant ou pendant la détection d'un indicateur clé s'écartant de ses valeurs ou plages de valeurs acceptables, indicateur clé s'écartant de ses valeurs ou plages de valeurs acceptables.

8. Procédé selon l'une quelconque des revendications 1 à 7, **caractérisé en ce que** le dispositif informatique (10) est un téléphone intelligent.

9. Procédé selon l'une quelconque des revendications 1 à 8, **caractérisé en ce que** le module d'évaluation (2) est configuré pour calculer les indicateurs clés sur la base d'au moins la pluralité de cycles d'enjambée.

10. Système d'analyse de la démarche d'un utilisateur comprenant :

     - un premier et un deuxième article de chaussure avec une pluralité de premiers et de deuxièmes capteurs de pression (1.1, 1.2, 1.3, 1.4, 1.5, 1.6, 1.7, 1.8) mesurant la pression qui y est appliquée et générant des signaux de sortie ; un premier et un second article de chaussure avec une pluralité de pression qui y est appliquée et générant des signaux de sortie ;
- un module d'évaluation respectif (2) intégré dans chaque article de chaussure (1), le module d'évaluation comportant une mémoire (4), une batterie (3), un dispositif de commutation configuré pour déterminer une capacité restante de la mémoire (4) et/ou un niveau de charge de la batterie (3), et un dispositif d'alarme (3) configuré pour déterminer un niveau de charge de la batterie (3).

et/ou un niveau de charge de la batterie (3), ainsi qu'un émetteur, le module d'évaluation (2) recevant les signaux de sortie des capteurs de pression (1.1, 1.2, 1.3, 1.4, 1.5, 1.6, 1.7, 1.8) ; et
- un dispositif informatique (10) avec un récepteur pour recevoir les données transmises par le module d'évaluation (2) ; et module d'évaluation (2) ;

dans lequel, lorsque la capacité restante de la mémoire (4) et/ou un niveau de charge de la batterie (3) du module d'évaluation (2) est suffisant, le module d'évaluation (2) est configuré pour :

   - enregistrer, pendant une pluralité de cycles d'enjambée, la pression appliquée à chaque capteur de pression (1.1, 1.2, 1.3, 1.4, 1.5, 1.6, 1.7, 1.8) pour obtenir une courbe correspondante de pression en fonction du temps pour chaque capteur (1.1, 1.2, 1.3, 1.4, 1.5, 1.6, 1.7, 1.8), la courbe correspondante étant déterminée à partir des signaux de sortie ;
   - déterminer un temps initial (ti) pour chacun des cycles d'enjambée sur la base des courbes ;
   - segmenter chaque courbe à chacun des temps initiaux (ti) ;
   - superposer les courbes segmentées d'au moins un capteur (1.1, 1.2, 1.3, 1.4, 1.5, 1.6, 1.7, 1.8) ;
   - calculer des indicateurs clés d'enjambée (SKI) basés sur les courbes segmentées superposées ; et courbes segmentées superposées ; et
   - transmettre des indicateurs clés d'enjambée (SKI) au dispositif informatique (10) ; et lorsque le dispositif de commutation détecte que la capacité restante de la mémoire (4) et/ou un niveau de charge de la batterie (3) du module d'évaluation (2) est inférieur à un seuil prédéterminé, le module d'évaluation (2) est configuré pour :
   transmettre les courbes de pression des cycles d'enjambée au dispositif de calcul (10) ; et le dispositif de calcul (10), à la réception de la pluralité de courbes de pression de cycles d'enjambée supplémentaires, est configuré pour :

   - enregistrer la courbe correspondante de pression en fonction du temps pour chaque capteur de pression (1.1, 1.2, 1.3, 1.4, 1.5, 1.6, 1.7, 1.8) ;
   - déterminer un temps initial (ti) pour chacun des cycles d'enjambée sur la base des courbes ;
   - segmenter chaque courbe à chacun des temps initiaux (ti) ;
   - superposer les courbes segmentées d'au moins un capteur (1.1, 1.2, 1.3, 1.4, 1.5, 1.6, 1.7, 1.8) ; et
   - calculer les indicateurs clés d'enjambée (SKI) sur la base des courbes segmentées superposées, et

dans lequel les valeurs ou plages de valeurs acceptables sont préétablies ou définies sur la base de valeurs moyennes ou de référence du cycle précédent, pour chaque indicateur clé d'enjambée (SKI), et lorsque l'un des indicateurs clés d'enjambée (SKI) s'écarte de ses valeurs ou plages de valeurs acceptables, un message d'avertissement s'affiche, valeurs ou gammes de valeurs acceptables, un signal est transmis au dispositif informatique dispositif informatique (10), et

dans lequel le module d'évaluation (2) est configuré pour générer un nouvel indicateur clé basé sur les variations persistantes détectées des courbes avant ou pendant la détection d'un indicateur clé d'enjambée s'écartant de sa valeur acceptable. avant ou pendant la détection d'un indicateur clé d'enjambée s'écartant de ses valeurs ou plages de valeurs acceptables. valeurs ou plages de valeurs acceptables.

**11.** Système selon la revendication 10, **caractérisé en ce que** chaque article de chaussure (1) comprend huit capteurs de pression (1.1, 1.2, 1.3, 1.4, 1.5, 1.6, 1.7, 1.8) répartis sur une partie de l'article de chaussure faisant face à la plante du pied et adaptés pour mesurer toute pression comprise entre 0,1 et 7 bars.

FIG 1

## FIG 2

## FIG 3

FIG 4

FIG 5

FIG 6